# EUROPÄISCHE PATENTSCHRIFT

(11) **EP 2 414 822 B1**
(45) Veröffentlichungstag und Bekanntmachung des Hinweises auf die Patenterteilung: **15.06.2016**
(21) Anmeldenummer: 10713601.2
(22) Anmeldetag: 30.03.2010
(51) Int. Cl.: G08B 17/117, G08B 29/18, G01N 27/414, G01N 33/00

(54) **SELEKTIVER DETEKTOR FÜR KOHLENMONOXID**
SELECTIVE DETECTOR FOR CARBON MONOXIDE
DÉTECTEUR SÉLECTIVE DE MONOXYDE DE CARBONE

(30) Priorität: 31.03.2009 DE 102009015121
(43) Veröffentlichungstag der Anmeldung: 08.02.2012
(73) Patentinhaber: Siemens Schweiz AG, 8047 Zürich (CH)
(72) Erfinder: FLEISCHER, Maximilian, 85635 Höhenkirchen (DE); HOEFER, Ulrich, CH-6300 Zug (CH); POHLE, Roland, 85570 Herdweg (DE); STEGMEIER, Stefan, 81543 München (DE)
(74) Vertreter: Maier, Daniel Oliver
(86) Internationale Anmeldenummer: PCT/EP2010/054144
(87) Internationale Veröffentlichungsnummer: WO 2010/112476

(56) Entgegenhaltungen:
- WO-A1-2005/103667
- DE-A1-102004 019 638
- DE-A1-102005 033 226
- DE-A1-102006 046 225
- US-A- 4 792 433
- SIEMONS M ET AL: "Preparation and gas sensing properties of nanocrystalline La-doped CoTiO3" SENSORS AND ACTUATORS B, ELSEVIER SEQUOIA S.A., LAUSANNE, CH LNKD- DOI:10.1016/J.SNB.2006.01.049, Bd. 120, Nr. 1, 14. Dezember 2006 (2006-12-14), Seiten 110-118, XP025112388 ISSN: 0925-4005 [gefunden am 2006-12-14]
- LEU M ET AL: "EVALUATION OF GAS MIXTURES WITH DIFFERENT SENSITIVE LAYERS INCORPORATED IN HYBRID FET STRUCTURES" SENSORS AND ACTUATORS B, ELSEVIER SEQUOIA S.A., LAUSANNE, CH LNKD- DOI:10.1016/0925-4005(93)01128-Q, Bd. B18, 1. Januar 1994 (1994-01-01), Seiten 678-681, XP000861740 ISSN: 0925-4005
- IVANOV P ET AL: "Towards a micro-system for monitoring ethylene in warehouses" SENSORS AND ACTUATORS B, ELSEVIER SEQUOIA S.A., LAUSANNE, CH LNKD- DOI:10.1016/J.SNB.2005.06.064, Bd. 111-112, 11. November 2005 (2005-11-11), Seiten 63-70, XP025329011 ISSN: 0925-4005 [gefunden am 2005-11-11]
- LAMPE U ET AL: "GasFET for the detection of reducing gases" SENSORS AND ACTUATORS B, ELSEVIER SEQUOIA S.A., LAUSANNE, CH LNKD- DOI:10.1016/J.SNB.2005.06.041, Bd. 111-112, 11. November 2005 (2005-11-11), Seiten 106-110, XP025329018 ISSN: 0925-4005 [gefunden am 2005-11-11]

## Beschreibung

Die Erfindung betrifft einen selektiven Detektor für Kohlenmonoxid, bevorzugt für Innenraumanwendungen, sowie ein Verfahren zum Betrieb eines solchen Detektors.
Die Detektion von CO (Kohlenmonoxid) in Luft ist für eine Reihe von Applikationen in Gebäuden von hohem Interesse. Einerseits ist CO geruchlos, aber gleichzeitig hochgiftig. So gilt für CO gilt derzeit ein MAK-Wert (=maximale Arbeitsplatz-Konzentration) von nur 30ppm. Wenn nun Feuerungsanlagen oder Kamine in einem Raum vorhanden sind, kann bei ungünstiger Verbrennung, d.h. bei Luftmangel, Kohlenmonoxid in einer Konzentration von mehreren Vol.-% im Rauchgas auftreten. Ist das Rauchrohr undicht oder gerät Kaminabluft durch ungünstige Druckverhältnisse - beispielsweise durch einen schlecht ziehenden Kamin oder den Betrieb einer Dunstabzugshaube in der Nähe - in den Raum, können sich toxische CO-Konzentrationen bilden. Diese können in ungünstigsten Umständen ausreichend sein, gesundheitliche Beeinträchtigungen bis hin zum Tod durch Vergiftung zu bewirken. So gibt beispielsweise das Center for Disease Control and Prevention in "Carbon Monoxide Poisoning: Fact Sheet" für die USA eine Zahl von jährlich
500 Toten durch CO-Vergiftung an.
Ein weiteres Interessensgebiet für die Detektion von CO ist dadurch gegeben, dass bei Schwelbränden massiv CO gebildet wird. Neben der Vergiftungsgefahr für Personen kann das Gas daher auch als Brandleitgas genutzt und daher seine Detektion auch zur frühzeitigen Erkennung von Brandsituationen eingesetzt werden.
Daher besteht ein hoher Bedarf an CO-Überwachungsgeräten, die kontinuierlich die Raumluft auf das Auftreten von CO überwachen. Für die Gebrauchsfähigkeit derartiger Geräte ist eine verlässliche Warnung ohne das Auftreten von Fehlalarmen wichtig. Das bedeutet, dass ein CO-Detektor das Kohlenmonoxid detektieren können muss, auch wenn bestimmte andere Gase in der Umgebung ebenfalls vorhanden sind. Solche Gase sind typischerweise Geruchsgase, beispielsweise flüchtige organische Kohlenwasserstoffe (VOC), Alkohole, die beim Alkoholkonsum oder aus Putzmitteln in die Luft gelangen, Luftfeuchte und aus der Außenluft eindringend oxidierende Gase (NO₂, O₃). Auch Schwankungen der Luft-Temperatur dürfen keine Fehlalarme auslösen.

Aus der US 4 792 433 ist eine Anordnung zum Messen von Kohlenmonoxid bekannt, wobei diese einen Kohlenmonoxid-Sensor mit einer zweiten Schicht zur Kohlenmonoxidmessung und einem Referenzsensor mit einer ersten Schicht zur Störgasmessung, beispielsweise Alkohol, umfasst. Beiden Schichten umfassen als Katalysatoren Pt, Pd, Rh oder Ag. Die Messungen des Kohlenmonoxids erfolgt bei Temperaturen von 100°C oder höher.

Aus Siemons et al., "Preparation and gas sensing of nanocrystalline La-doped CoTiO3", Sensors and Actuators B120 (2006) 110-118, ist bekannt, dass mittels der nanokristallinen Struktur CoTio₃:La dotiert mit Metallen Ethanol detektierbar ist. Aus P. Ivanov et al., "Towards a micro-system for monitoring ethylene in warehouses", Sensors and Actuators B111-112 (2005) 63-70, ist ein Micro-Sensor Array zum selektiven Messen von Ethylen, Ethanol und Ammoniak bekannt.

Ein bekannter Aufbau zur Detektion von Gasen ist ein GasFET, d.h. ein für die Gasdetektion ausgestalteter Feldeffekttransistor-Aufbau. Der GasFET weist an seinem Gate eine besondere gassensitive Schicht auf. An der gassensitiven Schicht entsteht durch eine gasinduzierte Änderung der Elektronenaustrittsarbeit ein zusätzliches Potential in der Größenordung von typischerweise 10-100 mV, welches als zusätzliche Gatespannung auf den Transistor wirkt und gemessen werden kann. Aus Leu et al., "Evaluation of gas mixtures with different sensitive layers incorporated in hybrid FET structures", Sensors and Actuators V18-19 (1994) 678-681, ist ein GasFET zur Messung der Gase Wasserstoff, Ammoniak, Stickstoffdioxid und Kohlenmonoxid bei Raumtemperatur (25°C) bekannt. Die DE 10 2006 046225 A1 offenbart eine Vorrichtung zur Erfassung mindestens eines Bestandteils in Luft, insbesondere Luftfeuchte, Kohlendioxid, dem Menschen schädigende Schadstoffe, Geruchsstoffe, gekennzeichnet durch mindestens einen gasempfindlichen Feldeffekttransistor mit mindestens einem gasempfindlichen Gate zur Erfassung des Bestandteils. Aus der WO 2005/103667 A1 ist ein FET-basierter Gassensor bestehend aus einer gassensitiven Schicht und einer Referenzschicht bekannt, wobei sich die Austrittsarbeitsänderung auf Zielgase nicht, die Austrittsarbeitsänderung auf nicht zu detektierende Gase sich jedoch in der Summe eliminieren. Aus der DE 10 2004 019 638 A1 ist ein FET-basierter CO-Sensor bekannt, der auf einem wenig katalytisch aktiven Grundmaterial, beispielsweise Galliumoxid, basiert, das mit einer katalytisch aktiven Edelmetalldispersion (z. B. Pt oder Pd) zur chemischen Aktivierung versehen wird. Durch die katalytische Aktivierung wird der Sensor wesentlich sensitiver gegenüber CO. Aus der US 2007/0181426 A1 ist ebenfalls ein FET-basierter CO-Sensor bekannt. Dieser basiert auf einem Metalloxid als Grundmaterial und einem darauf vorgesehenen Oxidationskatalysator. Aus der gleichen Schrift ist bekannt, den Sensor zur Detektion von Alkoholen oder Wasserstoff zu verwenden.

Diese CO-Sensoren sind erheblich querempfindlich gegenüber Lösemitteln wie Ethanol. Bei Verwendung alkoholhaltiger Reinigungsmittel in einem Gebäude sowie durch verschütteten Alkohol aus Getränken können Alkoholkonzentrationen bis zu mehreren 100 ppm auftreten. Diese bewirken die gleiche Signalhöhe beim Sensor wie die zu detektierende CO-Konzentration von 30 ppm. Es können also durch Ethanol leicht Fehlmessungen bewirkt werden.

Es ist bekannt, Aktivkohlefilter vor einem Sensorelement vorzusehen. Die Aktivkohlefilter absorbieren Ethanol und lassen CO passieren. Allerdings wird bei längerer Anwesenheit von Ethanol der Filter gesättigt, woraufhin er auch Ethanol passieren lässt. Daher ist eine verlässliche Filterung von Ethanol auch mit Aktivkohlefilterung nicht gewährleistet.

Aufgabe der vorliegenden Erfindung ist es, einen Kohlenmonoxid-Sensor anzugeben, der die obigen Nachteile vermeidet bzw. Probleme löst. Der Sensor soll insbesondere Kohlenmonoxid verlässlich detektieren können, auch wenn Alkohole in der Umgebungsluft vorhanden sind. Eine weitere Aufgabe besteht in der Angabe eines Betriebsverfahrens für einen solchen Sensor.

Diese Aufgabe wird durch einen Kohlenmonoxid-Sensor mit den Merkmalen von Anspruch 1 gelöst. Hinsichtlich des Verfahrens besteht eine Lösung in einem Betriebsverfahren mit den Merkmalen von Anspruch 9.

Die Erfindung basiert auf der Erkenntnis, dass bei Verwendung von metalloxidbasierten Sensorschichten die Reaktion auf CO und die Reaktion auf Ethanol auf unterschiedlichen Wirkmechanismen beruhen. So ist die Reaktion auf CO eine Redoxreaktion, die auf der Anwesenheit dahingehend katalytisch aktiver Zentren bewirkt wird. Die katalytisch aktiven Zentren können beispielsweise durch eine Katalysatordispersion oder katalytisch aktive Nebengruppen-Metalloxide in oder auf der metalloxidbasierten Sensorschicht dargestellt werden. Ethanol wiederum reagiert auch an derartigen katalytischen Zentren, benötigt diese Zentren aber nicht unbedingt. Die Reaktion auf Ethanol funktioniert auch auf Basis einer Wechselwirkung der die Alkohole kennzeichnenden -OH-Gruppe mit einem katalytisch nicht aktiven Metalloxid.

Die erfindungsgemäße Anordnung zur Detektion von Kohlenmonoxid weist eine erste, katalytisch inaktive, gassensitive Schicht zur Detektion von Konzentrationen von Ethanol auf. Die erste gassensitive Schicht ist dabei bevorzugt ausgestaltet, Konzentrationen von weniger als 1000 ppm Ethanol, insbesondere weniger als 100 ppm Ethanol detektieren zu können. Weiterhin weist die Anordnung eine zweite, katalytisch aktive, gassensitive Schicht zur Detektion von Konzentrationen von Kohlenmonoxid von weniger als 1000 ppm auf. Die Anordnung ist ausgestaltet, mittels der gassensitiven Schichten bei Beaufschlagung mit Kohlenmonoxid und/oder Ethanol wenigstens zwei verarbeitbare Signale zu erzeugen.

Die Anordnung weist also im Unterschied zu beispielsweise der US 2007/0181426 A1 zwei Sensorschichten auf. Dabei ist die erste gassensitive Schicht nicht nur gestaltet, Ethanol zu detektieren, sondern ist katalytisch inaktiv, detektiert also vorteilhaft CO wesentlich weniger stark als die zweite gassensitive Schicht.

Für die erste und zweite gassensitive Schicht kommen jeweils verschiedene Umsetzungen in Frage. So kann die zweite gassensitive Schicht ein Oxid eines Nebengruppenmetalls aufweisen. Beispiele hierfür sind La₂O₃, CeO₂, Mn₂O₃, MoO₃, TiO₂, V₂O₅. Dabei ist zweckmäßig das Oxid des Nebengruppenmetalls der Hauptbestandteil der Schicht. Bevorzugt besteht die Schicht soweit technisch möglich aus dem Oxid des Nebengruppenmetalls. Oxide von Nebengruppenmetallen sind soweit katalytisch aktiv, dass sie eine Detektion von Kohlenmonoxid bei Konzentrationen kleiner als 1000 ppm, insbesondere sogar kleiner als 100 ppm zulassen. Insbesondere ist die Sensitivität ausreichend, eine Überschreitung einer Alarmschwelle im Bereich des MAK-Werts von CO, also beispielsweise 30 ppm, zuzulassen.

Eine weitere Möglichkeit des Aufbaus der zweiten gassensitiven Schicht besteht darin, ein Oxid eines Hauptgruppenmetalls zu verwenden. Die erste gassensitive Schicht weist dann ein Oxid eines Hauptgruppenmetalls auf, beispielsweise Ga₂O₃, SnO₂, In₂O₃ oder Al₂O₃. Dabei ist zweckmäßig das Oxid des Hauptgruppenmetalls der Hauptbestandteil der zweiten gassensitiven Schicht. Bevorzugt besteht die Schicht soweit technisch möglich aus dem Oxid des Hauptgruppenmetalls. Oxide von Hauptgruppenmetallen sind soweit katalytisch inaktiv, dass sie auf CO nur bei Konzentrationen von weit mehr als 100 ppm reagieren. Um eine Sensitivität für CO zu erreichen, wird die zweite gassensitive Schicht mit einer Dispersion eines Katalysatormaterials versehen. Dafür können beispielsweise Platin oder Palladium verwendet werden. Ebenfalls können die bereits vorgenannten Oxide von Nebengruppenmetallen verwendet werden, also beispielsweise La₂O₃, CeO₂, Mn₂O₃, MoO₃, TiO₂, V₂O₅.

Eine dritte Möglichkeit für den Aufbau der zweiten gassensitiven Schicht besteht darin, dass die zweite gassensitive Schicht ein katalytisch aktives Metall aufweist. Dabei ist zweckmäßig das katalytisch aktive Metall der Hauptbestandteil der zweiten gassensitiven Schicht. Bevorzugt besteht die Schicht soweit technisch möglich aus dem katalytisch aktiven Metall.

Eine Möglichkeit für den Aufbau der ersten gassensitiven Schicht besteht darin, ein Oxid eines Hauptgruppenmetalls zu verwenden. Die erste gassensitive Schicht weist dann ein Oxid eines Hauptgruppenmetalls auf. Dabei ist zweckmäßig das Oxid des Hauptgruppenmetalls der Hauptbestandteil der ersten gassensitiven Schicht. Bevorzugt besteht die Schicht soweit technisch möglich aus dem Oxid des Hauptgruppenmetalls. Oxide von Hauptgruppenmetallen sind soweit katalytisch inaktiv, dass sie auf CO nur bei Konzentrationen von weit mehr als 100 ppm reagieren. Für den hier gedachten Zweck sind sie deshalb praktisch unsensitiv auf CO. Sie reagieren aber dennoch auf die Anwesenheit von Ethanol oder anderen Alkoholen.

Zweckmäßig ist es, wenn die Anordnung Mittel zur Auswertung der Signale der Schichten umfasst, die ausgestaltet sind, eine Ermittlung der Kohlenmonoxid-Konzentration unter Korrektur des Einflusses von Alkoholmolekülen auf die Messung durchzuführen. Konkret kann beispielsweise durch die zweite gassensitive Schicht die Konzentration von Kohlenmonoxid ermittelt werden. Die zweite gassensitive Schicht wird dabei aber auch von Ethanol oder anderen Alkoholen in der Umgebungsluft beeinflusst. Es ergibt sich also ein Summensignal, das Signalanteile beider Stoffe wiedergibt. Durch die erste gassensitive Schicht wird die Konzentration von Ethanol oder anderen Alkoholen ermittelt. Da die erste gassensitive Schicht nicht oder nur sehr geringfügig von CO beeinflusst wird, ist in ihrem Signal kein Signalanteil von CO enthalten. Beispielsweise durch eine Berechnung, beispielsweise eine Linearkombination beider Signale, kann die Konzentration von Ethanol oder anderen Alkoholen aus dem Summensignal der zweiten gassensitiven Schicht herausgerechnet werden und es verbleibt ein Signal, das die Konzentration von CO wiedergibt. Die Mittel umfassen zweckmäßig eine Elektronik, beispielsweise in Form eines Mikroprozessors, zur Signalaufnahme, Speicherung und Auswertung und beispielsweise auch zur Weitergabe der gewonnenen Information an andere Stellen. Bei dieser Konfiguration kann vorteilhaft auch eine Look-Up-Table eingesetzt werden, bei der die Messsignale für diverse Kombinationen der Alkohol und CO-Konzentrationen abgelegt sind, um die CO-Konzentration zu ermitteln. Diese Variante weist Vorteile bei nichtlinearen Zusammenhängen der Signale mit der Gaskonzentration auf.

Besonders vorteilhaft ist es, wenn der Sensor, der durch die erfindungsgemäße Anordnung gebildet wird, auf einem GasFET-Aufbau basiert. Dafür sind dann bevorzugt zwei getrennte Feldeffekttransistoraufbauten vorhanden. Jeder der Aufbauten ist mit einer der beiden gassensitiven Schichten auf seinem Gate versehen. Das Signal der GasFET-Aufbauten beruht auf einer durch Gase bewirkten Änderung der Austrittsarbeit.

Besonders vorteilhaft daran ist, dass das Signal bereits bei Raumtemperatur ausreichend ist, um eine Gasdetektion zu ermöglichen. Ebenfalls von besonderem Vorteil ist es, wenn für den GasFET-Aufbau ein sog. SGFET-Aufbau, d.h. ein Suspended-Gate-Aufbau oder ein CCFET, d.h. ein Capacitively Controlled FET, verwendet wird. Beide zeichnen sich durch ihren hybriden Aufbau aus, d.h. das gasempfindliche Gate und der eigentliche Transistor werden getrennt hergestellt und durch eine geeignete Technologie miteinander verbunden. Dadurch wird es möglich, zahlreiche Materialien als gassensitive Schicht in den Transistor einzubringen, deren Herstellbedingungen nicht mit denen beispielsweise der Siliziumtechnologie kompatibel sind. Dies trifft insbesondere auf Metalloxide zu, die in Dick- oder Dünnschichttechnologie mit anschließenden Hochtemperaturprozessen aufgebracht werden können.

In einer vorteilhaften Ausgestaltung der Erfindung kann die Anordnung einen weiteren Sensor enthalten, der zur Bestimmung der Luftfeuchte ausgestaltet ist. Damit ist es möglich, eine weitere Korrektur der ermittelten CO-Konzentration durchzuführen. Dadurch wird die Genauigkeit der CO-Messung erhöht, da Schwankungen der Luftfeuchte die ermittelten CO- und Ethanolsignale beeinflussen.

Bevorzugt ist die Anordnung ausgestaltet, dass der Betrieb und die Auslesung wenigstens der ersten gassensitiven Schicht bei Raumtemperatur geschehen. Mit anderen Worten wird hier kein Heizelement eingesetzt. Bei der zweiten Schicht ist es vorteilhaft, ein Heizelement vorzusehen.

Für den Betrieb des Heizelements gibt es zwei Möglichkeiten. Die erste Möglichkeit besteht darin, die zweite Schicht konstant auf einer leicht gegenüber der Raumtemperatur erhöhten Temperatur zu halten. Zweckmäßig hierfür sind Temperaturen von 100°C oder weniger, insbesondere 80°C oder weniger. Die zweite Möglichkeit besteht darin, die Sensoren intermittierend auf eine erhöhte Temperatur aufzuheizen und den eigentlichen Messbetrieb bei Raumtemperatur erfolgen zu lassen. Beispielsweise kann die zweite Schicht auf 150-200°C für einen Zeitraum von 5 Minuten aufgeheizt werden, wobei auf die 5 Minuten dann ein Messbetrieb von beispielsweise 1-10 Tagen folgt. Bevorzugt beträgt das Verhältnis der Dauer der Heizperiode zur Dauer der heizungslosen Messperiode weniger als 50 %, insbesondere weniger als 1 %. Bevorzugt werden die Sensorschichten grundsätzlich bei Temperaturen von weniger als 200°C betrieben.

Wenn die Sensoren mit thermischer Aktivierung betrieben werden, kann es je nach Ausführungsart nach dem Prozess des thermischen Aktivierens bis zu wenigen Stunden dauern, bis sich bei Raumtemperatur wieder der stabile Nullpunkt eingestellt hat. Um die eingeschränkte Messgenauigkeit in dieser Phase zu überbrücken kann das Sensorsystem aus 2 Sensorpaaren bestehen, bei denen abwechselnd eines im stabilen Messbetrieb bei Raumtemperatur gefahren wird und das andere die thermische Aktivierung und Stabilisierung durchläuft. Alternativ zur vollständigen Verdoppelung der Sensoranordnung ist es auch möglich, neben dem einzelnen Aufbau mit der bevorzugt unbeheizten ersten Sensorschicht zwei oder mehrere Aufbauten mit der zweiten Sensorschicht vorzusehen, die abwechselnd verwendet werden.

Typische Dicken der verwendeten Oxide liegen im Bereich 0,3 - 20 µm. Es können sowohl kompakte Schichten, hergestellt z. B. in Sputtertechnologie oder mit Sol-Gel-Verfahren in Spincoating oder Sprühverfahren verwendet werden, oder aber poröse Schichten, die vorzugsweise in Dickschichttechnologe hergestellt werden. Dabei bietet die Verwendung von porösen Schichten mit großer innerer Oberfläche und damit entsprechend höherer Reaktivität den Vorteil, dass hier generell höhere Signale erzielt werden können, die dann einfacher und präziser ausgelesen werden können.

Bevorzugte, jedoch keinesfalls einschränkende Ausführungsbeispiele für die Erfindung werden nunmehr anhand der Zeichnung näher erläutert. Dabei sind die Merkmale schematisiert dargestellt und sich entsprechende Merkmale sind mit gleichen Bezugszeichen markiert. Die Figuren zeigen dabei im Einzelnen
- Figur 1: einen CO-Sensor mit zwei GasFET-Aufbauten,
- Figur 2: einen Sensorsignal-Verlauf einer katalytisch aktiven Schicht
- Figur 3: einen Sensorsignal-Verlauf einer katalytisch inaktiven Schicht
- Figur 4: die Gassensitivität eine katalytisch inaktiven Schicht in Abhängigkeit von der Luftfeuchte (rH).

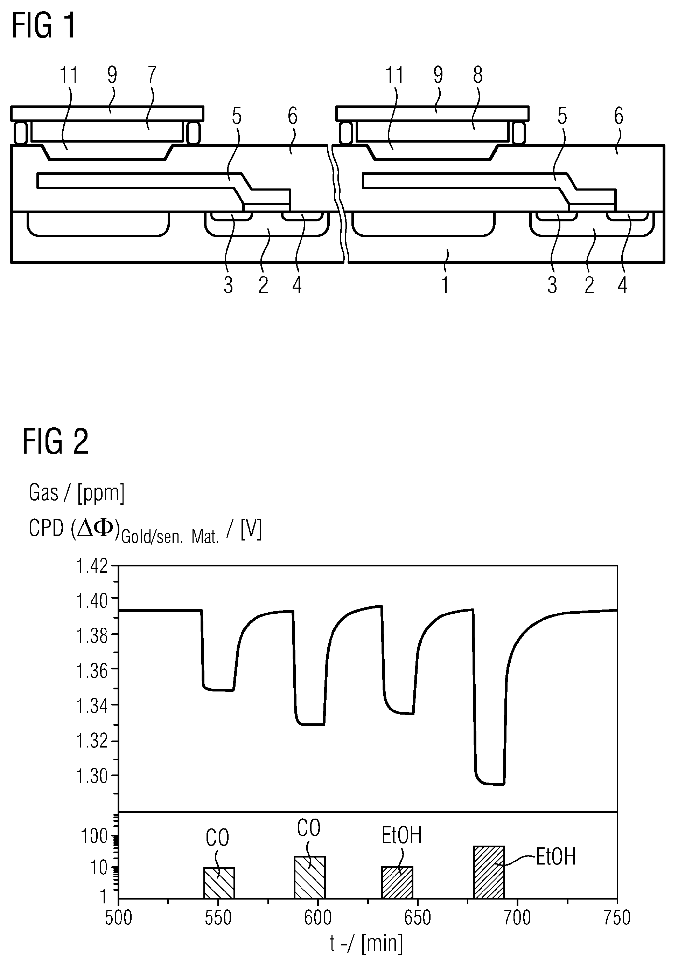

Der CO-Sensor gemäß der Figur 1 basiert auf einem p-SiliziumSubstrat 1. Der CO-Sensor weist dabei nebeneinander angeordnet zwei analog aufgebaute Feldeffektstrukturen auf. Die Feldeffektstrukturen sind dabei typischerweise weiter voneinander entfernt, als sich in Figur 1 maßstäblich darstellen ließe. Neben einer Fertigung auf einem Substrat lassen sich die Sensoren natürlich auch auf getrennten Substraten fertigen und nebeneinander auf einem externen Träger, beispielsweise einem Gehäuseboden oder eine Platine, anordnen.

Die Feldeffektstrukturen sind gleichartig aufgebaut. Sie weisen jeweils in einem n-Si-Bereich 2 einen Sourcebereich 3 und einen Drainbereich 4 auf. Darüber ist ein Floating Gate 5 vorgesehen innerhalb einer Passivierungsschicht 6 aus SiO₂. Das Floating Gate 5 ist von der Passivierungsschicht 6 bedeckt, wobei direkt über dem Floating Gate 5 ein verdünnter Bereich vorgesehen ist. Auf dem verdünnten Bereich kommt die jeweilige sensitive Schicht 7, 8. Die sensitiven Schichten 7, 8 werden dabei jeweils von einem eigenen Träger getragen, wobei dieser Träger so auf die Passivierungsschicht 6 aufgebracht wird, dass die sensitive Schicht 7, 8 zum Floating Gate 5 hinweist. Die sensitive Schicht 7, 8 bildet dann mit der Passivierungsschicht 6 - bedingt durch den verdünnten Bereich - einen Kanal 11, der den Gaszutritt zur sensitiven Schicht 7, 8 erlaubt.

Die erste gassensitive Schicht 7 besteht aus Galliumoxid (Ga₂O₃). Die zweite gassensitive Schicht 8 besteht ebenfalls aus Galliumoxid, wobei die zweite gassensitive Schicht 8 zusätzlich eine Katalysatordispersion aus Platin aufweist. In diesem Fall bestehen also die beiden gassensitiven Schichten 7, 8 aus dem gleichen Grundmaterial, und die zweite gassensitive Schicht 8 ist mit einem Zusatz versehen, um eine unterschiedliche Sensitivität hervorzurufen.

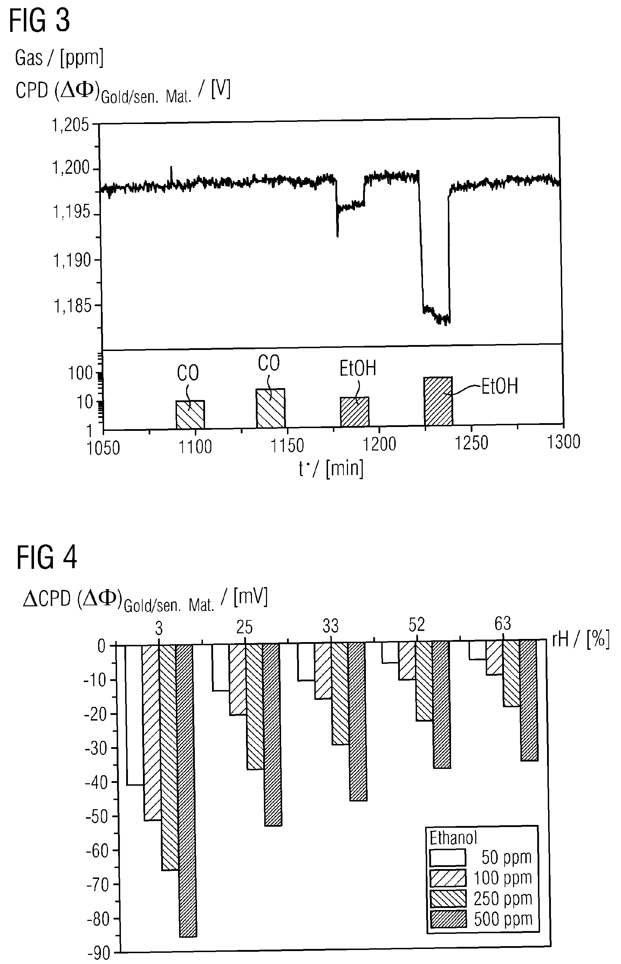

Den Figuren 2 und 3 kann der Verlauf des Sensorsignals der beiden Einzelsensoren des CO-Sensors gemäß Figur 1 entnommen werden. Dabei ist die erste gassensitive Schicht 7 als katalytisch wenig aktiv einzustufen. Dementsprechend zeigt sie gemäß der Figur 3 eine mäßige Sensitivität auf Ethanol (50 ppm), aber überhaupt keine sichtbare Reaktion auf CO (50 ppm). Die zweite gassensitive Schicht 8 ist durch den Platin-Zusatz hingegen als katalytisch aktiv einzustufen, was sich auch in der Sensorreaktion gemäß Figur 2 zeigt. Die zweite gassensitive Schicht 8 zeigt hier eine etwa 6-mal stärkere Reaktion auf beide Gase als die erste gassensitive Schicht 7. Insbesondere ist hier eine Reaktion auf CO zu beobachten, die in ihrer Stärke der Reaktion auf Ethanol in etwa entspricht.

Eine Linearkombination der Sensorsignale der beiden Einzelsensoren kann hier also verwendet werden, um aus den beiden Signalen der Einzelsensoren die Konzentration von CO zu errechnen. Besonders vorteilhaft ist hierbei, dass die erste gassensitive Schicht 7 so gut wie gar nicht auf CO reagiert, was die Berechnung der Konzentration von CO wesentlich genauer macht, als wenn beide Sensoren reagieren würden. Da die Reaktion von solchen Gassensoren üblicherweise keine lineare Funktion der Konzentration ist, kann die Genauigkeit der Messung durch die Verwendung einer Tabelle mit gespeicherten Wertepaaren aus Konzentration und Messsignal verbessert werden (look-up table).

Dabei kann vorteilhaft die erste gassensitive Schicht 7 bei auf Raumtemperatur betrieben werden, also ohne Beheizung. Dadurch wird Energie, die bei Festkörpersensoren meist durch die Beheizung benötigt wird, eingespart. Für batteriebetriebene Sensoren hat das neben einem vereinfachten Aufbau vor allem eine erhebliche Verlängerung der Lebensdauer bzw. Wartungsfreiheit zur Folge. Die zweite gassensitive Schicht 8 wird hingegen thermisch aktiviert. Dazu wird die zweite gassensitive Schicht 8 durch eine in Figur 1 nicht dargestellte Beheizung entweder kurzzeitig thermisch aktiviert (120-170°C für typischerweise 5 min) und dann bei Raumtemperatur gemessen oder auf einer konstanten Temperatur von beispielsweise 80°C gehalten. Insgesamt ergibt sich für den CO-Sensor ein sehr geringer Energieverbrauch, der einen Batteriebetrieb, drahtlose Sensornetzwerke oder auch den direkten Anschluss an Datenbusleitungen erlaubt.

Im bzgl. Figur 1 gegebenen Ausführungsbeispiel werden die beiden gassensitiven Schichten 7, 8 aus dem gleichen Grundmaterial aufgebaut. Eine alternative Ausführungsform ergibt sich, wenn die zweite gassensitive Schicht 8 stattdessen aus einem anderen Grundmaterial aufgebaut ist, beispielsweise aus einem katalytisch aktiven Metall wie Platin. Dieses ist bzgl. des Kohlenmonoxids als katalytisch aktiv anzusehen und ermöglicht ohne Zusatz eine CO-Detektion. Es werden hier also zwei verschiedene Grundmaterialien verwendet, aber die Katalysatordispersion kann unterbleiben.

Ein weiteres Sensitivitätsprofil, das in Figur 4 dargestellt ist, zeigt die Reaktion der ersten gassensitiven Schicht 7 auf Ethanol bei variierender Luftfeuchte. Figur 4 macht deutlich, dass eine schwankende Luftfeuchte eine gewisse Auswirkung auf das Sensorsignal auf Ethanol hat. Diesem Einfluss kann mit einem erweiterten CO-Sensor begegnet werden.

Der erweiterte CO-Sensor weist dabei drei einzelne Sensormodule auf. Das erste und zweite Sensormodul sind dabei gleichartig aufgebaut und entsprechen dem einfachen CO-Sensor gemäß dem vorangehend beschriebenen ersten Ausführungsbeispiel. Sie weisen also jeweils eine katalytisch aktive Sensorschicht und eine katalytisch inaktive Sensorschicht auf. Beide Sensormoduln weisen dabei eine gassensitive Schicht aus Zinnoxid, Sn02 auf. Diese Schicht kann hier als katalytisch inaktiv gelten, sodass dieser Sensor im Wesentlichen auf Ethanol reagiert, nicht aber auf CO. Weiterhin wird die gassensitive Schicht aus Zinnoxid auch durch Variationen der Luftfeuchte beeinflusst. Weiterhin weise beide Sensormoduln eine gassensitive Schicht aus MoO3 auf. Da Molybdän-Oxid als katalytisch aktiv anzusehen ist, reagiert diese neben Ethanol auch auf CO.

In diesem Ausführungsbeispiel sind die einfachen CO-Sensoren so aufgebaut, dass für jeden eine einzelne Beheizungsmöglichkeit, beispielsweise in Form einer metallischen Heizschleife für das gesamte Substrat 1, vorgesehen ist. In diesem Ausführungsbeispiel wird die thermische Aktivierung deshalb automatisch für beide Schichten eines Sensormoduls durchgeführt. Für die thermische Aktivierung werden die Schichten eines der Sensormodule für 5 Minuten beheizt und dann in der Folge 12 h unbeheizt gelassen. Dabei dauert es nach der Beheizung bis zu einigen Stunden, bis die Eigenschaften der Schicht sich wieder im Gleichgewicht befinden. Während der Einschwingzeit ist eine Messung erschwert. Deshalb wird beim erweiterten CO-Sensor die Beheizung der gassensitiven Schichten der Sensormodule immer abwechselnd vorgenommen. So wird das erste Sensormodul fünf Minuten lang beheizt und dann für 12 h nicht weiter verwendet. Während dieser ersten 12 h ist das zweite Sensormodul für die Messungen von CO und Ethanol zuständig. Nach Ablauf der 12 h wird das erste Sensormodul für die nächsten 12 h für die Gas-Messungen verwendet. Während dieser nächsten 12 h Zeit wird das zweite Sensormodul kurz beheizt und dann sich selbst überlassen, um eine Rückkehr ins Gleichgewicht zu ermöglichen. Nach dem Ablauf der nächsten 12 h wird wieder umgeschaltet, d.h. es wird wieder das zweite Sensormodul für die CO-Messung verwendet und das erste Sensormodul thermisch aktiviert.

Das dritte Sensormodul dient der Detektion der Luftfeuchte. Unter Verwendung aller Sensormoduln kann somit die CO-Konzentration unter Berücksichtigung von Einflüssen von Ethanol und anderen Alkoholen sowie der Luftfeuchte ermittelt werden. Dabei wird das Herausfiltern des Einflusses der Luftfeuchte analog zur Korrektur von Einflüssen von Ethanol durchgeführt. Somit ist ein Sensor gegeben, der ohne Gefahr von Fehlalarmen durch Alkohole - durch Putzmittel oder Getränke - eine Messung der Kohlenmonoxid-Konzentration im Bereich des MAK-Werts erlaubt.

Die Aufbauvarianten, die mit dem ersten und zweiten Ausführungsbeispiel beschrieben wurden, beziehen sich auf einen reinen CO-Sensor. Es ist selbstverständlich möglich, die Sensorkomponenten in eine größere Gruppe (Array) von Sensoren zu integrieren, die neben der Detektion von CO noch weitere Aufgaben durchführt. So könnte beispielsweise die CO-Detektion Teil eines Sensoraufbaus sein, der der Branderkennung dient. Die Erkennung von Kohlenmonoxid würde hierbei als Precursor-Gas zur Detektion von Schwelbränden dienen. Weiterhin könnte der CO-Sensor Teil einer Warnanlage sein, die auch andere Gase wie beispielsweise Erdgas, also Methan und Ethan, detektiert und ggf. ein Warnsignal abgibt.

## Patentansprüche

1. Anordnung zur Detektion von Kohlenmonoxid mit zwei Feldeffekttransistoraufbauten, aufweisend:
- eine erste, katalytisch inaktive, gassensitive Schicht (7) zur Detektion von Ethanol im Bereich eines ersten Gates des ersten Feldeffekttransistoraufbaus, wobei
- die erste gassensitive Schicht (7) ein Oxid eines Hauptgruppenmetalls aufweist,
- eine zweite, katalytisch aktive, gassensitive Schicht (8) im Bereich eines zweiten Gates des zweiten Feldeffekttransistoraufbaus, wobei
- die zweite gassensitive Schicht (8) ein Oxid eines Nebengruppenmetalls aufweist zur Detektion von Konzentrationen von Kohlenmonoxid von weniger als 1000 ppm,
wobei die Anordnung ausgestaltet ist, mittels der gassensitiven Schichten (7, 8) bei Beaufschlagung mit Kohlenmonoxid und/oder Ethanol wenigstens zwei verarbeitbare Signale zu erzeugen und die Anordnung Mittel zur Auswertung der Signale umfasst, wobei die Mittel ausgestaltet sind, eine Ermittlung der Kohlenmonoxid-Konzentration unter Korrektur des Einflusses von Ethanol auf die Messung durchzuführen.

2. Anordnung gemäß Anspruch 1, bei der die erste gassensitive Schicht (7) eines der Oxide Ga₂O₃, SnO₂, In₂O₃ oder Al₂O₃ aufweist.

3. Anordnung gemäß einem der vorangehenden Ansprüche, bei der die zweite gassensitive Schicht (8) eines der Oxide La₂O₃, CeO₂, Mn₂O₃, MoO₃, TiO₂, V₂O₅ aufweist.

4. Anordnung gemäß Anspruch 1, die einen weiteren Sensor zur Ermittlung der Luftfeuchte aufweist.

5. Verfahren zum Betrieb einer Anordnung gemäß einem der vorangehenden Ansprüche, bei dem die zweite gassensitive Schicht (8) zumindest zeitweise beheizt wird.

6. Verfahren gemäß Anspruch 5, bei dem die zweite gassensitive Schicht (8) zur thermischen Aktivierung für eine erste Zeitspanne beheizt wird und für eine zweite Zeitspanne unbeheizt betrieben und zur Messung verwendet wird, wobei das Verhältnis der Dauern der ersten Zeitspanne zur zweiten Zeitspanne weniger als 50 % beträgt.

7. Verfahren gemäß Anspruch 5, bei dem die zweite gassensitive Schicht (8) gleichbleibend beheizt wird.

8. Verfahren gemäß einem der Ansprüche 5 bis 7, bei dem die erste gassensitive Schicht (7) bei Raumtemperatur belassen wird.

## Claims

1. Arrangement for detecting carbon monoxide with two field-effect transistor structures, comprising:
- a first, catalytically inactive, gas-sensitive layer (7) for detecting ethanol in the region of a first gate of the first field-effect transistor structure, wherein
- the first gas-sensitive layer (7) includes an oxide of a main group metal,
- a second, catalytically active, gas-sensitive layer (8) in the region of a second gate of the second field-effect transistor structure, wherein
- the second gas-sensitive layer (8) includes an oxide of a subgroup metal for detecting concentrations of carbon monoxide of less than 1000 ppm,
wherein the arrangement is embodied to generate at least two processible signals by means of the gas-sensitive layers (7, 8) upon exposure to carbon monoxide and/or ethanol and the arrangement includes means for evaluating the signals, the means being embodied to determine the carbon monoxide concentration while correcting the effect of ethanol on the measurement.

2. Arrangement according to claim 1, in which the first gassensitive layer (7) includes one of the oxides Ga₂O₃, SnO₂, In₂O₃ or Al₂O₃.

3. Arrangement according to one of the preceding claims, in which the second gas-sensitive layer (8) includes one of the oxides La₂O₃, CeO₂, Mn₂O₃, MoO₃, TiO₂, V₂O₅.

4. Arrangement according to claim 1 which includes a further sensor for determining the relative humidity of the air.

5. Method for operating an arrangement according to one of the preceding claims, wherein the second gas-sensitive layer (8) is heated at least intermittently.

6. Method according to claim 5, wherein the second gas-sensitive layer (8) is heated for the purpose of thermal activation for a first time period and operated unheated for a second time period and is used for the measurement, the ratio of the duration of the first time period to the duration of the second time period being less than 50 %.

7. Method according to claim 5, wherein the second gas-sensitive layer (8) is heated constantly.

8. Method according to one of claims 5 to 7, wherein the first gas-sensitive layer (7) is left at room temperature.

## Revendications

1. Agencement de détection de monoxyde de carbone ayant deux structures de transistor à effet de champ, comportant :
- une première couche (7) inactive catalytiquement et sensible au gaz pour détecter de l'éthanol dans la région d'une première grille de la première structure de transistor à effet de champ, dans lequel
- la première couche ( 7 ) sensible au gaz comporte un oxyde d'un métal du groupe principal,
- une deuxième couche ( 8 ) active catalytiquement et sensible au gaz dans la région d'une deuxième grille de la deuxième structure de transistor à effet de champ, dans lequel
- la deuxième couche ( 8 ) sensible au gaz comporte un oxyde d'un métal d'un groupe auxiliaire pour la détection de concentration de monoxyde de carbone de moins de 1000 ppm,
l'agencement étant conformé pour produire au moyen des couches ( 7, 8 ) sensibles au gaz, lorsqu'il est soumis à du monoxyde de carbone et/ou à de l'éthanol, au moins deux signaux pouvant être traités et l'agencement comprend des moyens d'exploitation des signaux, les moyens étant conformés pour effectuer une détermination de la concentration de monoxyde de carbone avec correction de l'influence de l'éthanol sur la mesure.

2. Agencement suivant la revendication 1, dans lequel la première couche ( 7 ) sensible au gaz comporte l'un des oxydes Ga₂O₃, SnO₂, In₂O₃ ou Al₂O₃.

3. Agencement suivant l'une des revendications précédentes, dans lequel la deuxième couche ( 8 ) sensible au gaz comporte l'un des oxydes La₂O₃, CeO₂, Mn₂O₃, MoO₃, TiO₂, V₂O₅.

4. Agencement suivant la revendication 1, qui comporte un autre capteur de détermination de l'humidité de l'air.

5. Procédé pour faire fonctionner un agencement suivant l'une des revendications précédentes, dans lequel on chauffe au moins de temps en temps la deuxième couche ( 8 ) sensible au gaz.

6. Procédé suivant la revendication 5, dans lequel on chauffe pendant un premier laps de temps la deuxième couche ( 8 ) sensible au gaz pour l'activation thermique et on la fait fonctionner sans chauffage pendant un deuxième laps de temps et on l'utilise pour la mesure, le rapport des durées du premier laps de temps au deuxième laps de temps étant plus petit que 50 %.

7. Procédé suivant la revendication 5, dans lequel on chauffe constamment la deuxième couche ( 8 ) sensible au gaz.

8. Procédé suivant l'une des revendications 5 à 7, dans lequel on laisse la première couche ( 7 ) sensible au gaz à la température ambiante.
